# EUROPEAN PATENT SPECIFICATION

(11) **EP 3 061 073 B1**
(45) Date of publication and mention of the grant of the patent: **11.12.2019**
(21) Application number: 14805664.1
(22) Date of filing: 17.10.2014
(51) Int. Cl.: G06T 19/00

(54) **IMAGE VISUALIZATION**
BILDVISUALISIERUNG
VISUALISATION D'IMAGES

(30) Priority: 22.10.2013 US 201361893912 P
(43) Date of publication of application: 31.08.2016
(73) Proprietor: Koninklijke Philips N.V., 5656 AE Eindhoven (NL)
(72) Inventor: GOTMAN, Shlomo, NL-5656 AE Eindhoven (NL); ROMMAN, Zimam, NL-5656 AE Eindhoven (NL)
(74) Representative: Versteeg, Dennis John
(86) International application number: PCT/IB2014/065423
(87) International publication number: WO 2015/059613

(56) References cited:
- WO-A1-98/37517
- US-A1- 2009 034 684
- J. Charwat-Pessler, M. Musso, K. Entacher, B. Plank, P. Schuller-Götzburg, S. Tangl and A. Petutschnigg1: "Improving CT Image Analysis of Augmented Bone with Raman Spectroscopy", Journal of Applied Mathematics, vol. 2013 10 May 2013 (2013-05-10), pages 1-11, XP002738905, Retrieved from the Internet: URL:http://www.hindawi.com/journals/jam/20 13/271459/ [retrieved on 2015-04-23]

## Description

The following generally relates to image visualization and is described with particular application to computed tomography (CT). However, the following is also amenable to other imaging modalities such as magnetic resonance (MR), positron emission tomography (PET), single photon emission tomography (SPECT), ultrasound (US), and/or other imaging modalities.

A CT scanner generally includes an x-ray tube mounted on a rotatable gantry opposite a detector array across an examination region. The rotatable gantry and hence the x-ray tube rotate around the examination region. The x-ray tube emits radiation that traverses the examination region and is detected by the detector array. The detector array generates and outputs a signal indicative of the detected radiation. The signal is reconstructed to generate three dimensional volumetric image data.

For diagnostic reading, the reading clinician has viewed images using different visualization techniques. Generally, each technique provides some different information of a same anatomical area, highlighting and/or emphasizing different characteristics of the same anatomical area. Examples of such are two-dimensional (2D) visualization, three-dimensional (3D) visualization, applying various filters, changing various contrast/brightness settings, etc.

One approach includes creating multiple series of images using different techniques in advance (e.g., by the CT scanner). The user can then select and view or later select and view the multiple series of images on a computing system such as a Picture Archiving and Communication System (PACS) and/or other computing system. In another approach, a special software application is ran to create various views of the same anatomical area in real-time and to present them to the user "on-demand" and/or otherwise.

In both cases, to review multiple views, the user switches between the different views. Unfortunately, this may slow down the examination interpretation process and requires memorizing already seen views. Alternatively, the user displays various views simultaneously on the same screen. Unfortunately, this necessitates smaller size and reduced resolution of the displayed images, potentially becoming impractical with increased number of views to review.

Aspects described herein address the above-referenced problems and others.

The following describes a visualization approach for concurrently displaying multiple views (e.g., view region of interest viewports) of a same sub-region or anatomical area of image data using a different processing algorithm for each view. The multiple views are superimposed over a sub-portion of the image data. The multiple views do not overlap, and each of the views has a same geometry. Changes to certain visual characteristics of a view automatically changes the same visual characteristics in the other views. Changes to other visual characteristics of a view do not affect the display of the other views. This provides the user with a convenient way to review and interact with the viewing application, without the need to switch between different views of the same area and/or compromise image size and/or resolution.

In one aspect, a method as disclosed in claim 1 includes visually presenting a primary image in a main viewport of a display monitor. The primary image is displayed with a first processing algorithm. The method further includes visually presenting a primary region of interest over a sub-portion of the primary image. The primary region of interest identifies an area of interest in the primary. The method further includes visually presenting, concurrently with visually presenting a primary region of interest, at least one secondary region of interest over a different sub-portion of the primary image or outside of the primary image but within the main viewport. The at least one secondary region of interest shows the same area of interest as in the primary region of interest processed with a second different processing algorithm.

In another aspect, a computing system as disclosed in claim 8 includes a computer processor that executes instructions stored in computer readable storage medium. The instructions cause the computer processor to visually present a primary image in a main viewport of a graphical user interface displayed in a display monitor. The primary image is processed with a first processing algorithm. The instructions further cause the computer processor to visually present a primary region of interest over a sub-portion of the primary image. The primary region of interest identifies and shows an area of interest in the primary. The instructions further cause the computer processor to concurrently visually present at least one secondary region of interest over a different sub-portion of the primary image. The at least one secondary region of interest shows the same area of interest as in the primary region of interest processed with a second different processing algorithm.

In another aspect, a computer readable storage medium as disclosed in claim 13 is encoded with computer readable instructions. The computer readable instructions, when executed by a processer, cause the processor to: concurrently display at least two viewports over different sub-portions of image data displayed in a main view port. The at least two viewports show a same sub-region of the displayed image data, but processed with a different processing algorithm

The invention may take form in various components and arrangements of components, and in various steps and arrangements of steps. The drawings are only for purposes of illustrating the preferred embodiments and are not to be construed as limiting the invention.
FIGURE 1 schematically illustrates an imaging system including a computing system console with image visualization software.
FIGURE 2 illustrates an example graphical user interface which includes a main viewport image with multiple regions of interest, each regions of interest displaying the same anatomical area, but processed using a different processing algorithm.
FIGURE 3 schematically illustrates an example of the image visualization software of the imaging system of FIGURE 1.
FIGURE 4 schematically illustrates a variation of FIGURE 1 in which the computing system and the imaging system are separated apparatuses.
FIGURE 5 schematically illustrates an example of the image visualization software of the imaging system of FIGURE 4.
FIGURE 6 illustrates an example method for concurrently visualizing multiple views of a same sub-portion of an image, the data in each view processed using a different processing approach.

Spectral CT, unlike conventional non-spectral CT, captures spectral characteristics. That is, the resulting volumetric image data includes voxels that typically are represented in terms of gray scale values corresponding to relative radiodensity. The gray scale values reflect the attenuation characteristics of the scanned subject and/or object, and generally show structure such as anatomical structures within the scanned patient or object. Since the absorption of a photon by a material is dependent on the energy of the photon traversing the material, the detected radiation also includes spectral information, which provides additional information indicative of the elemental or material composition (e.g., atomic number) of the scanned material of the subject and/or object. A spectral CT scanner captures the above-noted spectral characteristics.

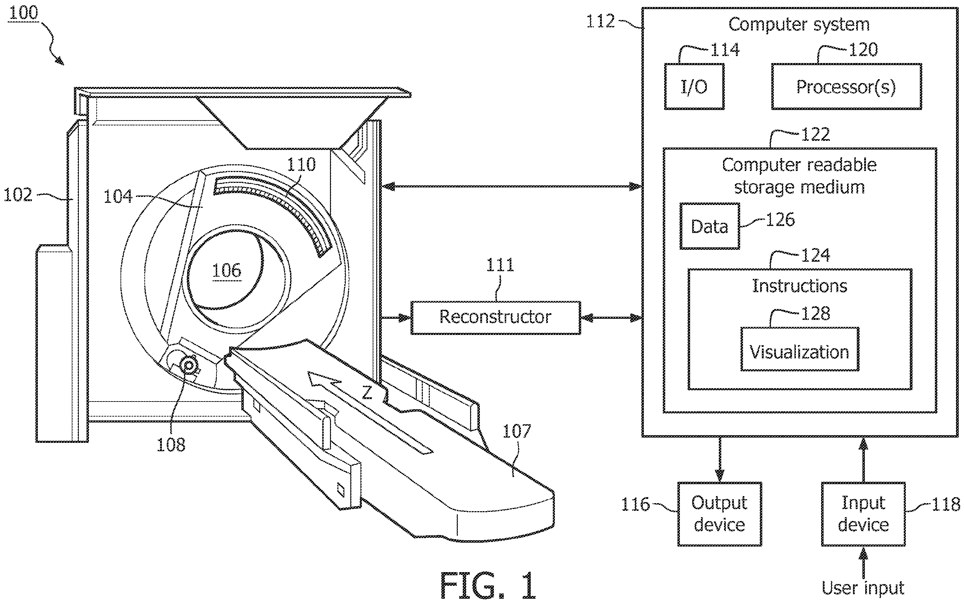

FIGURE 1 illustrates an imaging system 100 such as a computed tomography (CT) scanner. The illustrated CT imaging system 100 is configured for spectral CT imaging. In a variation, the imaging system 100 includes a non-spectral CT, a magnetic resonance (MR), a positron emission tomography (PET), an ultrasound (US), and/or other imaging modality. In another variation, the imaging system 100 includes a combination of one or more of a spectral CT, a non-spectral CT, an MR, a PET, a US, and/or other imaging modality.

The illustrated imaging system 100 includes a generally stationary gantry 102 and a rotating gantry 104. The rotating gantry 104 is rotatably supported by the stationary gantry 102 and rotates around an examination region about a longitudinal or z-axis. A subject support 107, such as a couch, supports an object or subject in the examination region. The subject support 107 is movable in coordination with performing an imaging procedure so as to guide the subject or object with respect to the examination region 106 for loading, scanning, and/or unloading the subject or object.

A radiation source 108, such as an x-ray tube, is rotatably supported by the rotating gantry 104. The radiation source 108 rotates with the rotating gantry 104 and emits radiation that traverses the examination region 106. In the illustrated embodiment, the radiation source 108 is a standard single x-ray tube. In another instance, the radiation source 108 is configured to be controllably switched between at least two different emission voltages (e.g., 80 kVp, 140 kVp, etc.) during scanning. In yet another instance, the radiation source 108 includes two or more x-ray tubes configured to emit radiation with different mean spectrums. In another instance, the radiation source 108 includes a combination of the above.

A radiation sensitive detector array 110 subtends an angular arc opposite the radiation source 108 across the examination region 106. The detector array 110 includes one or more rows of detectors that arranged with respect to each other along a z-axis direction, detects radiation traversing the examination region 106, and generates signals indicative thereof. The detector array 110 includes non-energy-resolving detectors and/or energy-resolving detectors.

A reconstructor 111 reconstructs the signals output by the detector array 110. This may include reconstructing one or more images for one or more different energy bins. Alternatively, this may include separately reconstructing signals from photosensors having different optical sensitivities. Alternatively, this may include decomposing a signal into Compton, photo-electric, and/or one or more K-edge components and reconstructing Compton, photo-electric, one or more K-edge, and/or combination images. The particular approach available depends on the spectral imaging configuration (i.e., single or multiple tubes, single or witching kVp, non-energy-resolving). Non-spectral imaging data can also be reconstructed.

A computing system 112 serves as an operator console. The console 112 allows an operator to control operation of the system 100. This includes selecting an imaging acquisition protocol(s), selecting a projection and/or image data processing algorithm(s), invoking scanning, invoking a visualization software application, interacting with an executing visualization software application, etc. The computing system 112 includes input/output (I/O) 114 that facilitates communication with at least an output device(s) 116 such as a display monitor, a filmer, etc., an input device(s) 118 such as a mouse, keyboard, etc.

The computing system 112 further includes at least one processor 120 (e.g., a central processing unit or CPU, a microprocessor, or the like) and a computer readable storage medium 122 (which excludes transitory medium), such as physical memory and/or other non-transitory memory. The computer readable storage medium 122 stores computer readable instructions 124 and data 126. The at least one processor 120 executes the computer readable instructions 124. The at least one processor 120 can also execute computer readable instructions carried by a signal, carrier wave, and other transitory (i.e., non-computer readable storage) medium.

The computer readable instructions 124 include at least visualization instructions 128. As described in greater detail below, the visualization instructions 128 visually present image data in a main viewport of a graphical user interface and one or more sub-viewports superimposed over different sub-regions of the visually presented image data. The sub-viewports include at least a primary region of interest (ROI) and one or more secondary ROIs. The primary ROI shows an area of interest in the primary image data, which is processed with a particular processing algorithm. The one or more secondary ROIs show the same area but with data processed using different processing algorithms.

The different processing algorithms include, but are not limited to, a polyenergetic X-Ray, a mono-energetic X-Ray, a relative material concentration, an effective atomic number, 2D/3D, and/or other processing algorithm. The other processing can be used to extract additional tissue information, enhance image quality, and/or increase the visualization of tissue/introduced contrast materials. This includes determining clinical values such as the quantification of contrast enhanced tissues, e.g., through an iodine map, generating a virtual non-contrast image from contrast enhanced image data, creating cine mode movies, displaying non-image data through charts, histograms, etc.

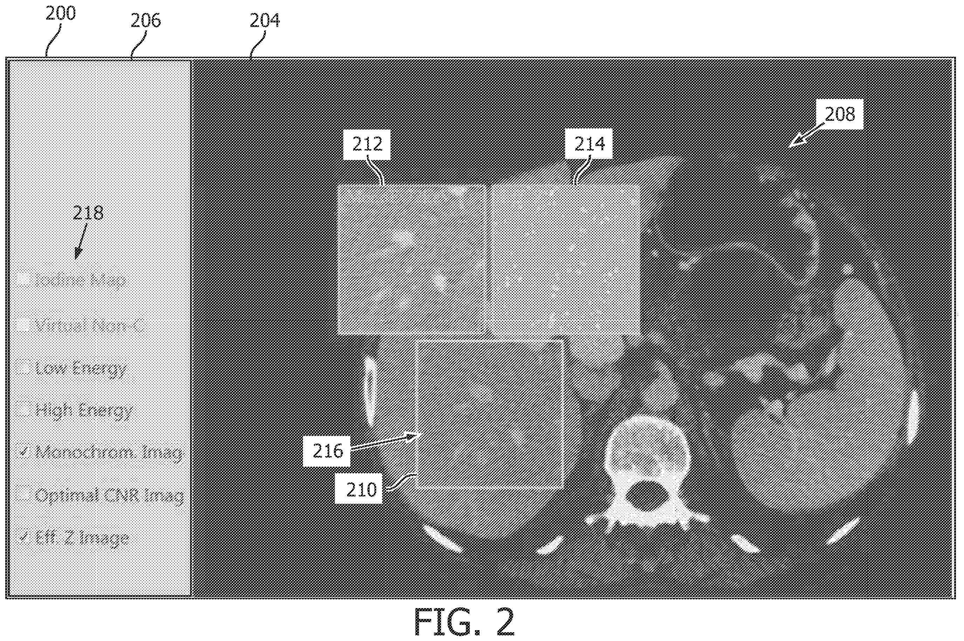

FIGURE 2 shows an example visualization which concurrently visually presents spectral image data of a same anatomical area of a primary image through multiple ROIs.

In FIGURE 2, a graphical user interface (GUI) 200 is displayed in a display monitor of the output devices 118. The GUI 200 includes a main viewport or image display region 204 and a menu display region 206. The image display region 204 visually presents a primary image 208 or slice of primary image data. The image display region 204 further visually presents a primary viewport ROI 210 and at least one secondary viewport ROI (two in the illustrated example, namely, a secondary ROI 212 and a secondary ROI 214), all superimposed over the primary image 208.

The primary ROI 210 defines an area or sub-set of pixels 216 of the primary image 208. The secondary ROI 212 and the secondary ROI 214 have a same size and a shape of the primary ROI 210 and include the same area or pixels with coordinates corresponding to the pixel coordinates in the primary ROI 210. However, the values of the pixels in the secondary ROI 212 and the secondary ROI 214 have intensity values from two different image data sets, each generated using a different processing algorithm relative to the primary image data and each other.

The menu display region 206 includes available secondary data sets 218 with data that can be presented in the secondary ROI 212 and the secondary 214. Selecting an available secondary data set from the sets 218 results in a secondary ROI being created and superimposed over the primary image data. The secondary ROI is visually presented such that it does not overlap the primary ROI or any other secondary ROI. Deselecting a selected available secondary data set from the sets 218 results in the corresponding secondary ROI being removed from the primary image data.

In the illustrated example, "monochrome. Imag" is selected (as can be seen from the check in the selection box corresponding to "monochrome. Imag") and "Eff Z image" is selected (as can be seen from the check in the selection box corresponding to "Eff. Z image"). The secondary ROI 212 corresponds to the image data for "monochrome. Imag" and secondary ROI 214 corresponds to the image data for "Eff. Z image"."Low Energy", "High Energy" and "Optimal CNR Image" are all selectable options but have not been selected. As such, secondary ROIs have not been created for them.

"Iodine Map" and "Virtual Non-C' are non-selectable options for the particular loaded data set. For example, the loaded data set is not an iodine contrast enhanced scan and no iodine map can be generated. Alternatively, an iodine contrast enhanced scan was performed but an iodine map has not been generated yet. In this instance, once an iodine map is generated, the "Iodine Map" will become a selectable option. In other embodiments, more or less, the same or different, etc. options are presented in the region 206. Generally, the options displayed in the sets 218 depend on the displayed image data.

Although FIGURE 2 is described in connection with spectral CT image data, it is to be understood that the ROIs 210, 212 and 214 may include non-spectral image data and/or spectral image data otherwise processed. With respect to spectral CT image data, other processing includes generating a mono-energetic image with a local optimized keV, generating a cine-mode movie with energy dependent images, energy adapting image brightness/contrast, creating non-image information in form of charts, histograms, etc.

With respect to a mono-energetic image with a local optimized keV, an optimum keV energy can vary according to the clinical question. For instance, this might include displaying data in an ROI in an energy that assures a best balance between iodine contrast and noise, energy that assures the best visualization of a certain body structure (such as the pancreatic duct), etc. The energy may be user adjustable through a soft control such as a graphical slider, a graphical knob, etc. Alternatively, the energy may be user adjustable through a physical control such as a keyboard button, mouse scroll wheel, etc.

With respect to cine mode, this includes scrolling through a set of mono-energetic images, each image just at a different keV. With respect energy adapting image brightness/contrast, mono-energetic images normally have different overall brightness and/or contrast (window and/or level) depending on keV value. As a result, the user needs to apply different window settings to achieve uniform viewing. In order to achieve uniform viewing, an ROI can automatically calculate and apply window and/or level settings according to the displayed mono-energetic image.

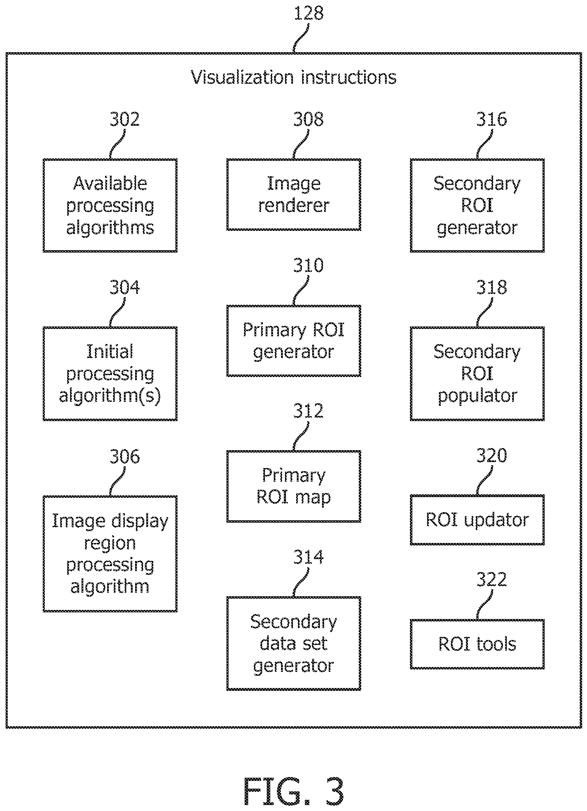

FIGURE 3 schematically illustrates an example of visualization instructions 128 in connection with FIGURE 1.

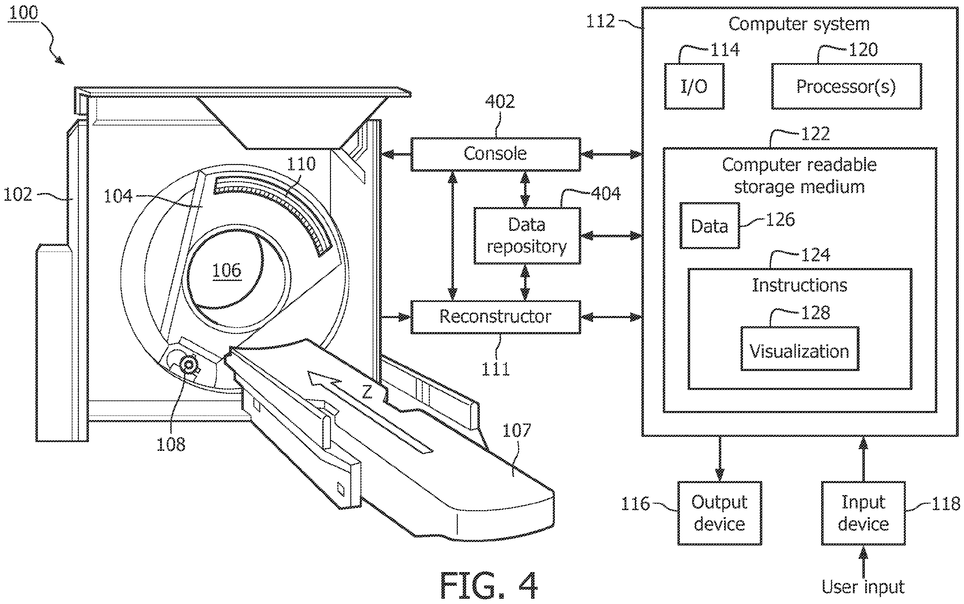

After scanning a subject, the spectral projection data can be stored in the computer readable storage medium 122 (FIGURE 1), reconstructed by the reconstructor 111 (FIGURE 1) (with the resulting image data be stored in the computer readable storage medium 122), conveyed to another computing system (e.g., the computing system of FIGURE 4), stored in other memory (e.g., the data repository of FIGURE 4), etc.

One or more of available processing algorithms 302 are used to process the projection and/or image data. Examples of such algorithms include, but are not limited to, energy specific processing, monochrome processing, effective Z (atomic number), etc. The processing algorithms 302 can be used on the fly on an on-demand basis when a particular processing algorithm is selected. Alternatively, the processing algorithms 302 can be used ahead of time with the processed data stored and accessible for subsequent visualization.

Initial processing algorithm(s) 304 identifies which processing algorithm(s) to initially employ. The initial processing algorithm(s) 304 may be predetermined and/or user selected. In one instance, the initial processing algorithm(s) 304 identifies only a single processing algorithm. Other processing algorithms can be later identified, e.g., in connection with reading images. In another instance, the initial processing algorithm(s) 304 identifies more than one processing algorithm and different sets of image data are initially processed.

Image display region processing algorithm 306 identifies the set of image data, where multiple processing algorithms have been used to process the data, to visually display in the main viewport or image display region 204 (FIGURE 2). The image display region processing algorithm 306 may be predetermined and/or user identified and/or selected. An image renderer 308 renders the identified set of image data in the image display region 204.

A primary region of interest (ROI) generator 310 creates the primary ROI 210 (FIGURE 2), which identifies the anatomical area of interest. In one instance, the primary ROI 210 is created through free hand drawing over or by placing a predetermined shape over the primary image 208. The primary ROI 210, generally, defines a closed perimeter or boundary which surrounds the subset of pixels and thus identifies the subset of pixels. The closed perimeter can take on various shapes including rectangle, square, circle, ellipse, irregular, and/or other shape.

The primary ROI 210 can be re-sized, moved so as to encompass a different sub-set of pixels of the primary image 208, rotated, and/or otherwise manipulated. The primary ROI 210 can also be removed from the primary image 208. More than one primary ROI can also be created and superimposed over the primary image 208. Activation can be in response to receiving an input signal from a control such as a physical button, a mouse click, touch of an area of a touch screen, etc. Termination can be invoked through the same and/or other control.

An ROI map 312 stores the size, shape, location (e.g., pixel coordinates), and/or other characteristic of the primary ROI 210.

A secondary data set menu generator 314 visually presents, in the menu display region 206, a menu or list 218 of the available processing algorithms 302 along with graphical (e.g., textual, pictorial, etc.) indicia that identifies the algorithms 302. The graphical indicia, in one instance, is selectable through at least one of the input devices 118 (e.g., a mouse), and selecting a particular graphical indicia identities another set of image data to visually present in the image display region in a secondary ROI. In one instance, the menu is automatically displayed. In another instance, the menu is displayed in response to a user input. In both or either instance, display of the menu can be toggled on and off.

A secondary ROI generator 316 generates at least one of the secondary viewport ROIs 212, 214, etc. in response to selection of particular graphical indicia in the menu. The number of secondary ROI's generated by the secondary ROI generator 316 is the same as the number of items selected from the menu. Each secondary ROI generated by the secondary ROI generator 316 is the same size and shape as the primary ROI 210. The secondary ROI generator 316 places each secondary ROI such that it does not overlap the primary ROI 210. The size, shape and location of the primary ROI 210 is obtained from the primary ROI map 312.

A secondary ROI populator 318 populates each secondary ROI 212, 214, etc. The pixels in each secondary ROI 212, 214, etc. have the same coordinates as the pixels in the primary ROI 210. The coordinates of the pixels in the primary ROI 210 is obtained from the primary ROI map 312. However, the pixels in each secondary ROI 212, 214, etc. include intensity values from the image data sets corresponding to the different image data set for the selected available processing algorithms 302.

A ROI updator 320 updates the information in the primary and secondary ROIs 212, 214, etc. For example, if the operator re-sizes the primary ROI 210, the ROI updator 320 automatically updates the secondary ROI(s). Another manipulation that automatically affects the secondary ROIs includes zoom. Generally, any manipulation that changes the sub-set of pixels in the primary ROI is automatically made to the secondary ROI(s). In one instance, this ensures the same sub-region of the subject is visually presented in each of the ROIs.

ROI tools 322 provide individual ROI tools for the ROIs 210, 212, 214, etc. For example, the ROI tools 322 allow the user to change window/level settings in one ROI without affecting the window/level settings in the other ROIs. Continuing with this example, this allows for setting different window/level settings for one of more of the primary ROI 210 and the secondary ROIs 212, 214, etc. Of course, a same window/level and/or other setting can be used in connection with two or more of the ROIs 210, 212, 214, etc. Another nonlimiting example of an individual tool is the energy level for a mono-energetic image.

FIGURE 4 schematically illustrates a variation of FIGURE 1 in which the computing system 112 is a separate apparatus from the imaging system 100.

In this variation, the imaging system 100 further includes a console 402. The console 402 includes a human readable output device such as a monitor or display and an input device such as a keyboard and mouse. Software resident on the console 402 allows the operator to interact with the scanner 100 via a graphical user interface (GUI) or otherwise. This includes selecting an imaging acquisition protocol(s), selecting a reconstruction algorithm(s), invoking scanning, invoking a visualization software application, etc.

The computing system 112 can receive projection data and/or image data to process from the imaging system 100 (the console 402 and/or the reconstructor 111), a data repository 404, another imaging system, and/or other device. An example of suitable data repository 404 is a picture archiving and communication system (PACS), a radiology information system (RIS), a hospital information system (HIS), an electronic medical record (EMR), a database, a server, an imaging system, and/or other data repository.

In this embodiment, the instructions 124 may also include a reconstructor. In this manner, the computing system 112 can reconstruct volumetric image data. In configurations in which the computing system 112 receives processed projection and/or image data for all of the available processing algorithms 302, the available processing algorithms 302 can be omitted.

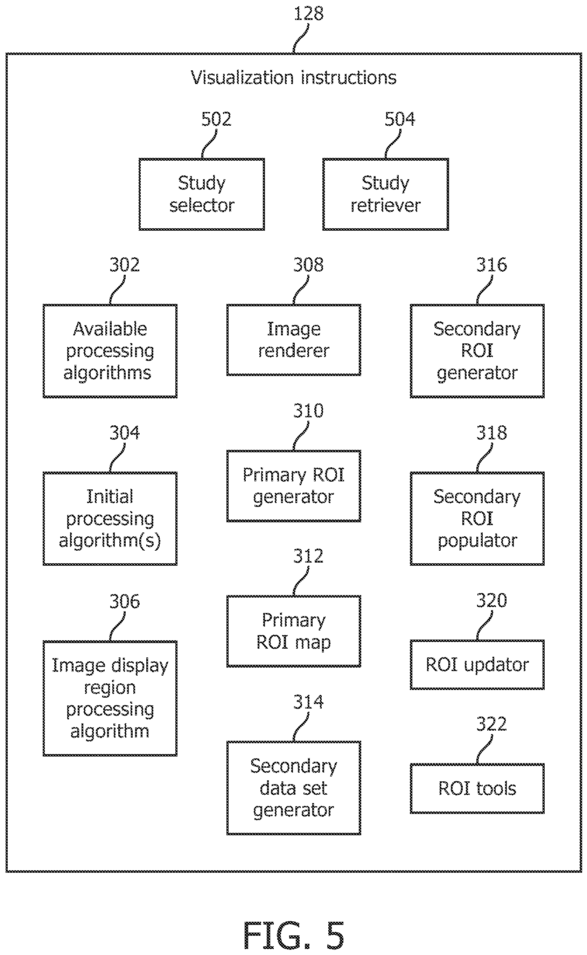

FIGURE 5 schematically illustrates an example of visualization instructions 128 in connection with FIGURE 4.

In this example, the visualization instructions 128 include those in FIGURE 3 with the addition of at least a study selector 502 and a study retriever 504. The study selector 502 visually presents a list of available studies to load. The studies can be stored on the imaging system 100, the data repository 404, in the data 126, and/or other device. The study selector 502 selects an available study to load in response to receiving an input from an input device 118. The data retriever 504 retrieves the selected study. The retrieved selected study may include projection and/or image data.

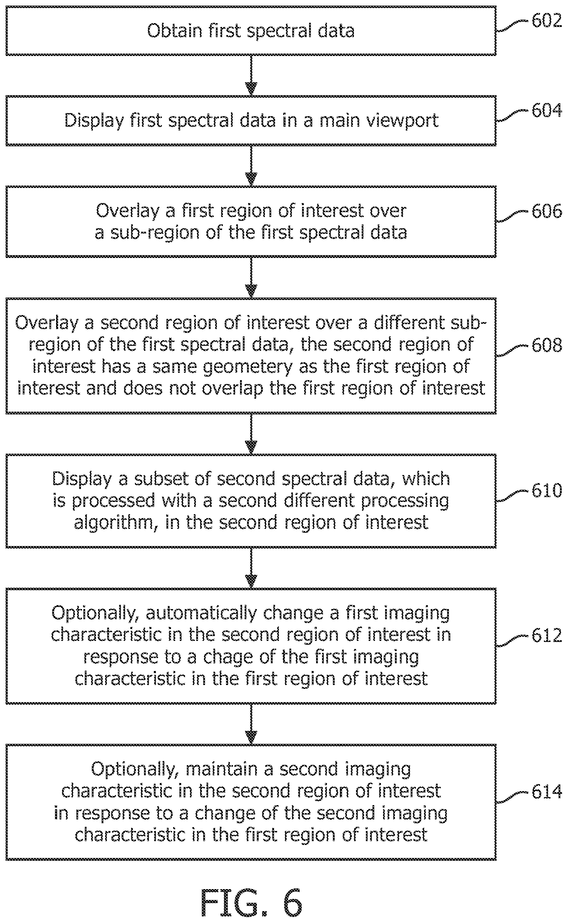

FIGURE 6 illustrate an example method. The illustrated method is describe in connection with different processed spectral image data.

It is to be appreciated that the ordering of the acts in the method is not limiting. As such, other orderings are contemplated herein. In addition, one or more acts may be omitted and/or one or more additional acts may be included.

At 602, first spectral data, created by processing obtained spectral projection and/or image data with a first processing algorithm, is obtained. Alternatively, non-spectral data can be obtained.

At 604, the first spectral data is visually displayed in an image viewport of a GUI visually presented via a display monitor. Likewise, non-spectral data can be obtained instead.

At 606, a first ROI viewport identifying a sub-region of interest of the first spectral data is overlaid over the first spectral data.

At 608, a second ROI viewport is overlaid over the first spectral data, where the second region of interest has a same geometry as and does not overlap the first region of interest. Additional ROI viewports may also be overlaid as such. The second and/or the additional viewports can be automatically and/or manually positioned.

At 610, a subset of second spectral data, created by processing the obtained data with a second different processing algorithm, corresponding to the same area defined by the first region of interest is displayed in the second region of interest. The additional ROI viewports will include the same area defined by the first region of interest processed using other processing algorithms.

At 612, optionally, a first imaging characteristic is changed in the first region of interest, which causes the same change to the second region of interest. Examples of the first imaging characteristic include zoom, pan, re-sizing the first region of interest, etc.

At 614, optionally, a second imaging characteristic is changed in the first or the region of interest, without causing the same change to the second or first region of interest. Examples of the second imaging characteristic include a change in the energy range of the of the image data, a changed in a filter applied to the image data, inclusion or removal of ancillary information such as a histogram, etc.

The above acts may be implemented by way of computer readable instructions, encoded or embedded on computer readable storage medium, which, when executed by a computer processor(s), cause the processor(s) to carry out the described acts. Additionally or alternatively, at least one of the computer readable instructions is carried by a signal, carrier wave or other transitory medium.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. An image visualisation method, comprising:
visually presenting a primary image (208) in a main viewport (204) of a display monitor (116),
wherein the primary image is processed with a first processing algorithm for generating primary image data;
visually presenting a primary region of interest (210) over a sub-portion of the primary image,
wherein the primary region of interest identifies an area of interest in the primary image which is processed with the first processing algorithm; and
visually presenting, concurrently with visually presenting a primary region of interest, at least one secondary region of interest (212, 214) over a different sub-portion of the primary image,
wherein the at least one secondary region of interest shows the same area of interest as in the primary region of interest processed with a second different processing algorithm for generating secondary image data different from the primary image data, wherein a geometry of the at least one secondary region of interest is the same as a geometry of the primary region of interest and wherein the at least one secondary region of interest does not overlap the primary region of interest.

2. The method of claim 1, further comprising:
receiving a first input indicating a first change of interest to a first visual characteristic of the sub-portion in the primary region of interest; and
automatically changing, in response to the first input, the same first visual characteristic of the sub-portion in the at least one secondary region of interest.

3. The method of any of claims 1 to 2, further comprising:
receiving a second input indicating a second change of interest to a second visual characteristic of the sub-portion in the primary region of interest; and
maintaining, in response to the second input, the same second visual characteristic of the sub-portion in the at least one secondary region of interest.

4. The method of any of claims 1 to 3, wherein the at least one secondary region of interest does not overlap the primary region of interest.

5. The method of any of claims 1 to 4, further comprising:
visually presenting a menu including available processing algorithms for the data displayed in the at least one secondary region of interest.

6. The method of claim 5, further comprising:
creating the at least one secondary region of interest in response to receiving a first input selecting one of the available processing algorithms.

7. The method of any of claims 5 to 6, further comprising:
removing the at least one secondary region of interest in response to receiving a second input deselecting a selected one of the available processing algorithms.

8. A computing system (100), comprising:
a computer processor (120) that is configured to execute instructions (128) stored in computer readable storage medium (122), which is to cause the computer processor to:
visually present a primary image in a main viewport of a graphical user interface (200) displayed in a display monitor, wherein the primary image is processed with a first processing algorithm for generating primary image data;
visually present a primary region of interest over a sub-portion of the primary image, wherein the primary region of interest identifies and shows an area of interest in the primary image which is processed with the first processing algorithm; and
concurrently visually present at least one secondary region of interest over a different sub-portion of the primary image, wherein the at least one secondary region of interest shows the same area of interest as in the primary region of interest processed with a second different processing algorithm for generating secondary image data different from the primary image data, wherein a geometry of the at least one secondary region of interest is the same as a geometry of the primary region of interest and wherein the at least one secondary region of interest does not overlap the primary region of interest.

9. The computing system of claim 8, wherein the computing system is one or a component of an imaging system or a distinctly separate apparatus with respect to an imaging system.

10. The computing system of any of claims 7 to 9, wherein at least one of the first processing algorithm or the second different processing algorithm is a spectral processing algorithm.

11. The computing system of any of claims 7 to 10, wherein executing instructions is to cause the computer processor to further:
change a first visual characteristic of the sub-portion in the primary region of interest in response to receiving a first input indicating the first change; and
automatically change, in response to the change of the first visual characteristic, the same first visual characteristic of the sub-portion in the at least one secondary region of interest.

12. The computing system of any of claims 7 to 11, wherein executing instructions is to cause the computer processor to further:
change a first visual characteristic of the sub-portion in the primary region of interest in response to receiving a first input indicating the first change; and
maintain the same first visual characteristic of the sub-portion in the at least one secondary region of interest, in response to the change of the first visual characteristic in the sub-portion in the primary region of interest.

13. A computer readable storage medium encoded with one or more computer executable instructions, which, when executed by a processor of a computing system according to claim 8, causes the processor to perform the steps according to claim 1.

## Patentansprüche

1. Bildvisualisierungsverfahren, umfassend:
visuelles Darstellen eines Primärbildes (208) in einem Hauptansichtsfenster (204) eines Anzeigemonitors (116),
wobei das Primärbild mit einem ersten Verarbeitungslogarithmus zum Generieren von Primärbilddaten verarbeitet wird;
visuelles Darstellen einer Primärregion von Interesse (210) über einem Teilbereich des Primärbildes,
wobei die Primärregion von Interesse einen Bereich von Interesse in dem Primärbild identifiziert, das mit dem ersten Verarbeitungsalgorithmus verarbeitet wird; und
visuelles Darstellen, gleichzeitig mit visuellem Darstellen einer Primärregion von Interesse, mindestens einer Sekundärregion von Interesse (212, 214) über einen unterschiedlichen Teilbereich des Primärbildes,
wobei die mindestens eine Sekundärregion von Interesse den gleichen Bereich von Interesse zeigt, wie in der Primärregion von Interesse, verarbeitet mit einem zweiten unterschiedlichen Verarbeitungsalgorithmus zum Generieren von Sekundärbilddaten, die sich von den Primärbilddaten unterscheiden, wobei eine Geometrie der mindestens einen Sekundärregion von Interesse die gleiche ist, wie eine Geometrie der Primärregion von Interesse, und wobei die mindestens eine Sekundärregion von Interesse nicht mit der Primärregion von Interesse überlappt.

2. Verfahren nach Anspruch 1, weiter umfassend:
Empfangen einer ersten Eingabe, die eine erste Veränderung von Interesse zu einer ersten visuellen Eigenschaft des Teilbereichs in der Primärregion von Interesse angibt; und
automatisches Ändern, in Reaktion auf die erste Eingabe, der gleichen ersten visuellen Eigenschaft des Teilbereichs in der mindestens einen Sekundärregion von Interesse.

3. Verfahren nach einem der Ansprüche 1 bis 2, weiter umfassend:
Empfangen einer zweiten Eingabe, die eine zweite Veränderung von Interesse zu einer zweiten visuellen Eigenschaft des Teilbereichs in der Primärregion von Interesse angibt; und
Aufrechterhalten, in Reaktion auf die zweite Eingabe, der gleichen zweiten visuellen Eigenschaft des Teilbereichs in der mindestens einen Sekundärregion von Interesse.

4. Verfahren nach einem der Ansprüche 1 bis 3, wobei die mindestens eine Sekundärregion von Interesse die Primärregion von Interesse nicht überlappt.

5. Verfahren nach einem der Ansprüche 1 bis 4, weiter umfassend:
visuelles Darstellen eines Menüs, das verfügbare Verarbeitungsalgorithmen für die Daten beinhaltet, die in der mindestens einen Sekundärregion von Interesse angezeigt werden.

6. Verfahren nach Anspruch 5, weiter umfassend:
Erzeugen der mindestens einen Sekundärregion von Interesse in Reaktion auf Empfangen einer ersten Eingabe, die einen der verfügbaren Verarbeitungsalgorithmen auswählt.

7. Verfahren nach einem der Ansprüche 5 bis 6, weiter umfassend:
Entfernen der mindestens einen Sekundärregion von Interesse in Reaktion auf Empfangen einer zweiten Eingabe zum Aufheben eines ausgewählten einen der verfügbaren Verarbeitungsalgorithmen.

8. Rechensystem (100), umfassend:
einen Computerprozessor (120), der konfiguriert ist, Anweisungen (128) auszuführen, die in einem computerlesbaren Speichermedium (122) gespeichert sind, die den Computerprozessor veranlassen:
ein Primärbild in einem Hauptansichtsfenster einer grafischen Benutzerschnittstelle (200), das auf einem Anzeigemonitor angezeigt wird, visuell darzustellen, wobei das Primärbild mit einem ersten Verarbeitungsalgorithmus zum Erzeugen von Primärbilddaten verarbeitet wird;
eine Primärregion von Interesse über einem Teilbereich des Primärbildes visuell darzustellen, wobei die Primärregion von Interesse einen Bereich von Interesse in dem Primärbild identifiziert und zeigt, der mit dem ersten Verarbeitungsalgorithmus verarbeitet wird; und
gleichzeitig mindestens eine Sekundärregion von Interesse über einen unterschiedlichen Teilbereich des Primärbildes visuell darzustellen,
wobei die mindestens eine Sekundärregion von Interesse den gleichen Bereich von Interesse zeigt, wie in der Primärregion von Interesse, verarbeitet mit einem zweiten unterschiedlichen Verarbeitungsalgorithmus zum Generieren von Sekundärbilddaten, die sich von den Primärbilddaten unterscheiden, wobei eine Geometrie der mindestens einen Sekundärregion von Interesse die gleiche ist, wie eine Geometrie der Primärregion von Interesse, und wobei die mindestens eine Sekundärregion von Interesse nicht mit der Primärregion von Interesse überlappt.

9. Rechensystem nach Anspruch 8, wobei das Rechensystem ein oder eine Komponente eines Abbildungssystems ist oder eine deutlich getrennte Vorrichtung in Bezug auf ein Abbildungssystem.

10. Rechensystem nach einem der Ansprüche 7 bis 9, wobei der erste Verarbeitungsalgorithmus und/oder der zweite unterschiedliche Verarbeitungsalgorithmus ein Spektralverarbeitungsalgorithmus ist.

11. Rechensystem nach einem der Ansprüche 7 bis 10, wobei Ausführen von Anweisungen den Computerprozessor weiter zu veranlassen hat:
eine erste visuelle Eigenschaft des Teilbereichs in der Primärregion von Interesse in Reaktion auf Empfangen einer ersten Eingabe, die die erste Änderung angibt, zu ändern; und
automatisch, in Reaktion auf die Änderung der ersten visuellen Eigenschaft, die gleiche erste visuelle Eigenschaft des Teilbereichs in der mindestens einen Sekundärregion von Interesse zu ändern.

12. Rechensystem nach einem der Ansprüche 7 bis 11, wobei Ausführen von Anweisungen den Computerprozessor weiter zu veranlassen hat:
eine erste visuelle Eigenschaft des Teilbereichs in der Primärregion von Interesse in Reaktion auf Empfangen einer ersten Eingabe, die die erste Änderung angibt, zu ändern; und
die gleiche erste visuelle Eigenschaft des Teilbereichs in der mindestens einen Sekundärregion von Interesse in Reaktion auf die Änderung der ersten visuellen Eigenschaft in dem Teilbereich in der Primärregion von Interesse aufrecht zu erhalten.

13. Computerlesbares Speichermedium, das mit einer oder mehreren computerausführbaren Anweisungen kodiert ist, die, wenn sie von einem Prozessor eines Rechensystems nach Anspruch 8 ausgeführt werden, den Prozessor veranlassen, die Schritte nach Anspruch 1 durchzuführen.

## Revendications

1. Procédé de visualisation d'image, comprenant :
la présentation visuellement d'une image primaire (208) dans une fenêtre de travail principale (204) d'un écran d'affichage (116),
dans lequel l'image primaire est traitée avec un premier algorithme de traitement pour produire des données d'image primaire ;
la présentation visuelle d'une région primaire d'intérêt (210) sur une sous-portion de l'image primaire,
dans lequel la région primaire d'intérêt identifie une zone d'intérêt dans l'image primaire qui est traitée avec le premier algorithme de traitement ; et
la présentation visuelle, concurremment avec une présentation visuelle d'une région primaire d'intérêt, d'au moins une région secondaire d'intérêt (212, 214) sur une sous-portion différente de l'image primaire,
dans lequel l'au moins une région secondaire d'intérêt montre la même zone d'intérêt que dans la région primaire d'intérêt traitée avec un second algorithme de traitement différent pour produire des données d'image secondaire différentes des données d'image primaire, dans lequel une géométrie de l'au moins une région secondaire d'intérêt est la même qu'une géométrie de la région primaire d'intérêt et dans lequel l'au moins une région secondaire d'intérêt ne chevauche pas la région primaire d'intérêt.

2. Procédé selon la revendication 1, comprenant en outre :
la réception d'une première entrée indiquant un premier changement d'intérêt à une première caractéristique visuelle de la sous-portion dans la région primaire d'intérêt ; et
le changement automatique, en réponse à la première entrée, de la même première caractéristique visuelle de la sous-portion dans l'au moins une région secondaire d'intérêt.

3. Procédé selon l'une quelconque des revendications 1 à 2, comprenant en outre :
la réception d'une seconde entrée indiquant un second changement d'intérêt à une seconde caractéristique visuelle de la sous-portion dans la région primaire d'intérêt ; et
le maintien, en réponse à la seconde entrée, de la même seconde caractéristique visuelle de la sous-portion dans l'au moins une région secondaire d'intérêt.

4. Procédé selon l'une quelconque des revendications 1 à 3, dans lequel l'au moins une région secondaire d'intérêt ne chevauche pas la région primaire d'intérêt.

5. Procédé selon l'une quelconque des revendications 1 à 4, comprenant en outre :
la présentation visuelle d'un menu incluant des algorithmes de traitement disponibles pour les données affichées dans l'au moins une région secondaire d'intérêt.

6. Procédé selon la revendication 5, comprenant en outre : la création de l'au moins une région secondaire d'intérêt en réponse à la réception d'une première entrée sélectionnant l'un des algorithmes de traitement disponibles.

7. Procédé selon l'une quelconque des revendications 5 à 6, comprenant en outre :
l'enlèvement de l'au moins une région secondaire d'intérêt en réponse à la réception d'une seconde entrée désélectionnant l'un sélectionné des algorithmes de traitement disponibles.

8. Système informatique (100), comprenant :
un processeur informatique (120) qui est configuré pour exécuter des instructions (128) stockées dans un support de stockage lisible par ordinateur (122), qui doit amener le processeur informatique à :
présenter visuellement une image primaire dans une fenêtre de travail principale d'une interface utilisateur graphique (200) affichée sur un écran d'affichage, dans lequel l'image primaire est traitée avec un premier algorithme de traitement pour produire des données d'image primaire ;
présenter visuellement une région primaire d'intérêt sur une sous-portion de l'image primaire, dans lequel la région primaire d'intérêt identifie et montre une zone d'intérêt dans l'image primaire qui est traitée avec le premier algorithme de traitement ; et
présenter concurremment visuellement au moins une région secondaire d'intérêt sur une sous-portion différente de l'image primaire, dans lequel l'au moins une région secondaire d'intérêt montre la même zone d'intérêt que dans la région primaire d'intérêt traitée avec un second algorithme de traitement différent pour produire des données d'image secondaire différentes des données d'image primaire, dans lequel une géométrie de l'au moins une région secondaire d'intérêt est la même qu'une géométrie de la région primaire d'intérêt et dans lequel l'au moins une région secondaire d'intérêt ne chevauche pas la région primaire d'intérêt.

9. Système informatique selon la revendication 8, dans lequel le système informatique est un composant particulier ou un composant d'un système d'imagerie ou un appareil distinctement séparé par rapport à un système d'imagerie.

10. Système informatique selon l'une quelconque des revendications 7 à 9, dans lequel au moins l'un du premier algorithme de traitement ou du second algorithme de traitement différent est un algorithme de traitement spectral.

11. Système de calcul selon l'une quelconque des revendications 7 à 10, dans lequel l'exécution d'instructions sert à amener le processeur informatique à en outre :
changer une première caractéristique visuelle de la sous-portion dans la région primaire d'intérêt en réponse à la réception d'une première entrée indiquant le premier changement ; et
changer automatiquement, en réponse au changement de la première caractéristique visuelle, la même première caractéristique visuelle de la sous-portion dans l'au moins une région secondaire d'intérêt.

12. Système informatique selon l'une quelconque des revendications 7 à 11, dans lequel l'exécution d'instructions sert à amener le processeur informatique à en outre :
changer une première caractéristique visuelle de la sous-portion dans la région primaire d'intérêt en réponse à la réception d'une première entrée indiquant le premier changement ; et
maintenir la même première caractéristique visuelle de la sous-portion dans l'au moins une région secondaire d'intérêt, en réponse au changement de la première caractéristique visuelle dans la sous-portion dans la région primaire d'intérêt.

13. Support de stockage lisible par ordinateur codé avec une ou plusieurs instructions exécutables par ordinateur, qui, lorsqu'elles sont exécutées par un processeur d'un système informatique selon la revendication 8, amènent le processeur à effectuer les étapes selon la revendication 1.
